# EUROPEAN PATENT APPLICATION

(11) **EP 2 305 290 A1**
(43) Date of publication of application: **06.04.2011**
(21) Application number: 09012591.5
(22) Date of filing: 05.10.2009
(51) Int. Cl.: A61K 38/48, A61K 47/26, A61K 47/42

(54) **Salt-free composition comprising an intact Botulinum toxin complex**

(71) Applicant: Merz Pharma GmbH & Co. KGaA, 60318 Frankfurt am Main (DE)
(72) Inventor: Eisele, Karl-Heinz, Dr., 60385 Frankfurt am Main (DE); Vey, Martin, Dr., 35039 Marburg (DE)
(74) Representative: Ricker, Mathias

(57) **Abstract**

A salt-free pharmaceutical composition comprising an intact Botulinum toxin complex and at least one pharmaceutically acceptable excipient.

## Description

### FIELD OF THE INVENTION

The invention relates to a salt-free pharmaceutical composition comprising an intact Botulinum toxin complex and a pharmaceutically acceptable excipient.

### BACKGROUND OF THE INVENTION

Botulinum toxins have been used in clinical settings for the treatment of diseases or conditions caused by or associated with hyperactive cholinergic enervations of muscles or exocrine glands in a patient. Botulinum toxin type A was approved by the U.S. Food and Drug Administration in 1989 for the treatment of essential blepharospasm, strabismus and hemifacial spasm in patients over the age of twelve. Clinical effects of peripheral intramuscular botulinum toxin type A are usually seen within one week of injection. The typical duration of symptomatic relief (i.e. flaccid muscle paralysis) from a single intramuscular injection of botulinum toxin type A can be about three months.

Seven immunologically distinct botulinum neurotoxins have been characterized, these being respectively botulinum neurotoxin serotypes A, B, C 1, D, E, F and G each of which is distinguished by neutralization with serotype-specific antibodies. The different serotypes of botulinum toxin vary in the animal species that they affect and in the severity and duration of the paralysis they evoke. The botulinum toxins apparently bind with high affinity to cholinergic motor neurons, are translocated into the neuron and block the presynaptic release of acetylcholine.

Although all the botulinum toxins serotypes apparently inhibit release of the neurotransmitter acetylcholine at the neuromuscular junction, they do so by affecting different neurosecretory proteins and/or cleaving these proteins at different sites. Botulinum toxin A is a zinc endopeptidase which can specifically hydrolyze a peptide linkage of the intracellular, vesicle associated protein SNAP-25. Botulinum toxin type E also cleaves the 25 kilodalton (kD) synaptosomal associated protein (SNAP-25), but targets different amino acid sequences within this protein, as compared to botulinum toxin type A. Botulinum toxin types B, D, F and G act an vesicle-associated protein (VAMP, also called synaptobrevin), with each serotype cleaving the protein at a different site. Finally, botulinum toxin type C, has been shown to cleave both syntaxin and SNAP-25. These differences in mechanism of action may affect the relative potency and/or duration of action of the various botulinum toxin serotypes.

The molecular weight of the neurotoxic component of Botulinum toxin, for all seven of the known botulinum toxin serotypes, is about 150 kD. Said neurotoxic component is responsible for the therapeutic effect of Botulinum toxins.

Interestingly, the botulinum toxins are released by Clostridial bacterium as complexes comprising the 150 kD neurotoxic component along with associated non-toxin proteins. Thus, the botulinum toxin type A complex can be produced by Clostridial bacterium as 900 kD, 500 kD and 300 kD forms. Botulinum toxin types B and C, is apparently produced as only a 500 kD complex. Botulinum toxin type D is produced as both 300 kD and 500 kD complexes. Finally, botulinum toxin types E and F are produced as only approximately 300 kD complexes. The complexes (i.e. molecular weight greater than about 150 kD) are believed to contain a non-toxin hemaglutinin protein and/or a non-toxin and non-toxic nonhemaglutinin protein.

Pharmaceutical compositions comprising the Botulinum toxin complex are known in the art. Commercial formulations comprising the Botulinum toxin complex type A are known under the trade names BOTOX^{®} and DYSPORT^{®}. A composition comprising the Botulinum toxin complex type B is commercialized under the tradename MYOBLOC^{®}. These compositions are widely used for treating diseases or conditions caused by or associated with hyperactive cholinergic enervations of muscles or exocrine glands in a patient.

These pharmaceutical compositions comprise the Botulinum toxin complex together with a suitable protein stabilizer, typically HSA and a buffer, typically NaCl or a phosphate buffer. The pharmaceutical compositions are in lyophilized form and are obtained by reconstituting the lyophilized pharmaceutical composition in an isotonic sodium chloride solution as an injectable solution.

It was believed and emphasized that the associated non-toxin proteins (which along with the neurotoxic component constitute the respective Botulinum complex) provide stability against denaturation of the neurotoxic component and protection against the acidic environment of the intestinal tract.

It was furthermore believed that the known pharmaceutical compositions comprising the Botulinum toxin complex together with a stabilizer and a buffer, in particular the above-identified commercial pharmaceutical compositions, comprise the Botulinum toxin complex in *intact* form, i.e. in the form of the 150 kD neurotoxic component together with the complex proteins associated with said neurotoxic component. For example Botox^{®} is marketed as comprising the Botulinum toxin complex as a 900 kD particle, i.e. protein complex.

As will be outlined hereinafter, it was found by the present inventors that this is not the case, but that Botox^{®} comprises the Botulinum toxin complex in a disrupted or dissociated state. In other words, Botox^{®} prepared for injection as instructed by the manufacturer contains a mixture of free neurotoxic component and free bacterial complexing proteins.

US 5 969 077 relates to a pharmaceutical preparation comprising a frozen, dried sodium chloride-free preparation of botulinum toxin type B. According to the description, other salts, such as sodium phosphate are, however, part of said preparation.

The effect of certain excipients including sodium chloride on the stabilization of botulinum toxin A during lyphilization was also subject of a scientific publication in *"*Applied and Environmental Microbiology, 1992, p. 3426-3428*".*

### OBJECTS OF THE INVENTION

In view of the above finding it was the object of the present invention to provide a pharmaceutical composition comprising an *intact* Botulinum toxin complex in the form of the neurotoxic component and, associated therewith, the complex proteins, e.g. wherein said complex is kept stable and active during e.g. lyophilization, dilution and reconstitution procedures.

This object was achieved by the composition as claimed in the present application.

### SUMMARY OF THE INVENTION

The present invention relates to a salt-free pharmaceutical composition comprising an intact Botulinum toxin complex and at least one pharmaceutically acceptable excipient.

In one embodiment the excipient is selected from one or more members of the group consisting of a saccharide, a polysaccharide, a di-saccharide, a mono-saccharide, amylopectin, glucopyranose, starch, hydroxyethyl starch, hydroxymethyl starch, sucrose, PEG.

In another embodiment the excipient is selected from one or more members of the group consisting of albumin, human serum albumin, bovine serum albumin, recombinant albumin, cryoprotectant, preservative , analgesic and sterile distilled water.

In still another embodiment the botulinum toxin complex is selected from the group consisting of botulinum toxin types A, B, C1, D, E, F and G.

In a further embodiment the botulinum toxin is botulinum toxin type A.

In a still further embodiment the invention relates to a lyophilized or vacuum dried salt-free pharmaceutical composition consisting essentially of a high molecular weight polysaccharide and a botulinum toxin complex.

In one embodiment the preceding claims in lyophilized, vacuum dried or liquid injectable form.

In a further embodiment the invention relates to a method for stabilizing a Botulinum toxin complex against denaturation or aggregation, the method comprising the step of formulating a Botulinum neurotoxin complex without any salt.

In another embodiment the invention relates to a method for the preparation of a salt-free pharmaceutical composition as defined above comprising the admixture of a Botulinum toxin complex and at least one pharmaceutically acceptable excipient, wherein said Botulinum toxin complex is not subjected to lyophylisation prior to said step of admixing.

The invention also relates to a method of using the pharmaceutical composition according to the invention, or the pharmaceutical composition prepared by the method of the invention for the treatment of a disease or condition caused by or associated with hyperactive cholinergic enervation of muscles or exocrine glands comprising the step of local administration of said pharmaceutical composition to a patient in need thereof.

It also relates to a pharmaceutical composition as defined above, or a pharmaceutical composition prepared by the method as defined above for use in the treatment of a disease or condition caused by or associated with hyperactive cholinergic enervations of muscles or exocrine glands in a patient in need thereof.

It also relates to the use of said pharmaceutical composition, or the pharmaceutical composition prepared by said method for cosmetic treatment.

### DETAILED DESCRIPTION OF THE INVENTION

The invention relates to a salt-free pharmaceutical composition comprising an intact Botulinum toxin complex and at least one pharmaceutically acceptable excipient.

The term "pharmaceutical composition" as used herein refers to a formulation in which an active ingredient for use as a medicament or a diagnostic is contained or comprised. Such pharmaceutical composition may be suitable for diagnostic or therapeutic administration (i.e. by intramuscular or subcutaneous injection) to a human patient.

The pharmaceutical composition to be used herein may comprise the Botulinum toxin complex as the sole active component or may contain additional pharmaceutically active components.

Generally, the pharmaceutical composition of the present invention comprises Botulinum toxin complex in a quantity of about 6 pg to about 300 ng. However, also doses of up to 1000 ng may be administered, depending on the condition to be treated , the severity thereof and/or the serotype used.

It has been surprisingly found that within such a composition the Botulinum toxin complex can be kept intact, if the composition is salt-free. The term "salt-free" as used herein means that the composition of the invention is free of sodium chloride. In another embodiment "salt-free" means free of any ionic compounds that are the result of a neutralization reaction between acid and base, and which are composed of cations and anions, irrespective of whether it is an inorganic salt, such as NaCl, or an organic salt, e.g. sodium acetate.

The term "free" (in "salt-free") means that the concentration of salts is 10 µmolar or less.

The term "intact botulinum toxin complex" means that within the botulinum toxin complex the neurotoxic component is in direct physical contact with at least one clostridial complexing protein. In one embodiment at least 90% of the neurotoxic component is in direct physical contact with at least one clostridial complexing protein after reconstitution with a salt-free solution. In another embodiment at least 95% or at least 99% of the neurotoxic component is in direct physical contact with at least one clostridial complexing protein after reconstitution with a salt-free solution. For Botulinum toxin type A said (intact) complex has a molecular weight of at least 500 kD. In one embodiment, the Botulinum toxin complex of type A is present as a 500 kD complex.

The direct physical contact is usually a bonding through non-covalent interactions, elsewhere referred to as "associated with".

During the course of achieving the present invention it was surprisingly found that, performing the lyophilization and/or the subsequent reconstitution of Botulinum toxin complex in the absence of sodium chloride, or in the absence of any salt, preserves an intact Botulinum toxin complex and results in an increased stability of said Botulinum toxin complex. This allows the administration of same, in an intact state, to a patient. According to the teaching of the present invention, this effect can even be enhanced by omitting any lyophylisation step after purifying the Botulinum toxin complex and by formulating the isolated complex into a pharmaceutical composition simply by admixing same with at least one suitable excipient in the absence of salt in order to provide a pharmaceutical composition according to the present invention.

Thus, it was found that the stability of the complex in the pharmaceutical composition as claimed is increasing in the order "lyophilization with salt", "lyophilization without salt" and "no lyophilization at all", the best result being achieved with no lyophilization at all.

According to the teaching of the present invention, the Botulinum toxin complex may be produced essentially as described in the art (Molecular Biology, vol. 145 (2000), p. 27-39; WO 2006/133818), however, with the exception that exposure of Botulinum toxin to salts is being avoided and, in one embodiment, no step of lyophylisation is being performed.

In one embodiment the Botulinum complex is derived from "hall strain A" (ATCC 3502). The genome is approximately 3,886,916 bp in size, with a G+C content of approximately 28.2%. There exists also a plasmid of 16,344 bp, which is encompassed by this definition. In another embodiment also any modified Botulinum complex, wherein the native hall strain A botulinum complex was used as starting basis, is encompassed.

The pharmaceutical composition may be lyophilized or vacuum dried, reconstituted, or may prevail in solution. When reconstituted, in one embodiment the reconstituted solution is prepared adding sterile distilled water.

The composition as claimed comprises excipients. The term "excipient" refers to a substance present in a pharmaceutical composition other than the active pharmaceutical ingredient present in the pharmaceutical composition. An excipient can be a buffer, carrier, antiadherent, analgesic, binder, disintegrant, filler, diluent, preservative, vehicle, cyclodextrin and/or bulking agent such as albumin, gelatin, collagen, preservative, cryoprotectant and/or stabilizer.

"Stabilizing", "stabilizes" or "stabilization" means that the active ingredient, i.e., the Botulinum toxin complex in a reconstituted or aqueous pharmaceutical composition has greater than about 20%, 30%, 40%, 50%, 60%, 70%, 80%, 90%, and up to about 100% of the toxicity that the complex had prior to being incorporated into the pharmaceutical composition. Toxicity may be measured using methods known in the art, e.g. a conventional LD₅₀ assay or a hemidiaphragma assay.

The native non-toxic proteins of the Botulinum toxin complex are suitable to stabilize the neurotoxic component, however, further stabilizers might be added. Thus, it is envisaged by the present invention that, based on the present invention's teaching, it is now possible to produce salt-free Botulinum toxin complex free of any stabilizing components. Accordingly, in one aspect, the pharmaceutical composition of the present invention is free of stabilizing components, in particular, said composition is free of human or bovine serum albumin and/or free of low molecular weight stabilizers such as sugars, surfactants and/or stabilizing amino acids. However, at least for the pharmaceutical composition in liquid injectable form, typically when being reconstituted, care has to be taken that excipients are present that prevent or at least minimize the effect of hypotonicity. Typically said excipients are a saccharide, a polysaccharide, a di-saccharide, a mono-saccharide, amylopectin, glucopyranose, starch, hydroxyethyl starch, hydroxymethyl starch, sucrose, PEG, or mixtures of two or more thereof.

Examples for such stabilizers are gelatin or albumin, in one embodiment of human origin or obtained from a recombinant source. Proteins from non-human or non-animal sources may also be included. The stabilizers may be modified by chemical means or by recombinant genetics. In one embodiment of the present invention, it is envisaged to use alcohols, e.g., inositol or mannitol, as cryoprotectant excipients to stabilize proteins during lyophilization.

In another embodiment of the present invention, the stabilizer may be a non proteinaceous stabilizing agent comprising a hyaluronic acid or a polyvinylpyrrolidone (Kollidon), hydroxyethyl starch, alginate or a polyethylene glycol or any combination thereof. Said composition may also comprise mono-, di- or higher polysaccharides, such as glucose, sucrose or fructose.

Said hyaluronic acid, when present in the instant composition, is in one embodiment combined with the instant polypeptide of the invention in a quantity of 0.1 to 10 mg, especially 1 mg hyaluronic acid per ml in a 200 U/ml botulinum toxin solution.

The polyvinylpyrrolidone (Kollidon) when present in the instant composition, is combined with the Botulinum toxin complex in such a quantity to provide a reconstituted solution comprising 10 to 500 mg, especially 100 mg polyvinylpyrrolidone per ml in a 200 U/ml solution of the complex. In another embodiment reconstitution is carried out in up to 8 ml solution. This results in concentrations of down to 12.5 mg polyvinylpyrrolidone per ml in a 25 U/ml solution of the polypeptide of the invention.

In particular, it pertains to a formulation comprising a hydrophilic polymer, a mixture of a polyalcohol and a sugar, wherein the weight ratio of polyalcohol to sugar is from 2:1 to 5:1 (wt-%), a detergent, and wherein the formulation is free of stabilising proteins.

In one embodiment of the present invention, the hydrophilic polymer is selected from the group consisting of hyaluronic acid, polyvinylpyrollidone (PVP), copolymers of N-vinylpyrollidone, cellulose derivatives, Polyethyleneglycol (PEG), PEG / PPG block copolymers, homo- and copolymers of acrylic and methacrylic acid, polyurethanes, polyvinyl alcohol (PVA), polyvinylethers, maleic anhydride based copolymers, polyesters, vinylamines, polyethyleneimines, polyethyleneoxides, poly(carboxylic acids), polyamides, polyanhydrides, polyphosphazenes and mixtures thereof.

The term "polyalcohol" as used herein relates to a group of carbohydrate-based ingredients, which are employed to protect the protein against instability. The term "polyol" and "sugar alcohols" are used synonymously.

The term "sugar" as used herein relates to any monosaccharide, disaccharide and polysaccharide. The term "monosaccharide" as used herein relates to the basic units of carbohydrates. The term "disaccharide" within the scope of the present invention relates to carbohydrates composed of two monosaccharides. The term "polysaccharides" as used herein relates to repeating units of monosaccharides, wherein the monosaccharides are bound with glycosidic bonds.

In one embodiment of the instant invention the hydrophilic polymer employed is hyaluronic acid and the detergent employed is Polysorbate 80.

In one further embodiment of the present invention the hydrophilic polymer employed is hyaluronic acid and the detergent employed is Polysorbate 20.

In one further embodiment of the present invention the hydrophilic polymer employed is polyvinylpyroldone (PVP) and the detergent employed is Polysorbate 80.

In one further embodiment of the present invention, the composition comprises hyaluronic acid, mannitol, sucrose and Polysorbate 80.

In one further embodiment of the present invention, the composition claimed herein comprises hyaluronic acid, sucrose and Polysorbate 80.

In one further embodiment of the present invention, the composition claimed herein comprises polyvinylpyrollidone (PVP), mannitol, and Polysorbate 80.

In one further embodiment of the present invention, the composition claimed herein comprises polyvinylpyrollidone (PVP), mannitol, sucrose and Polysorbate 80.

In one embodiment the composition further comprises sucrose or human serum albumin or both, in still another embodiment the ratio of human serum albumin to sucrose is about 1:5. In another embodiment, said human serum albumin is recombinant human serum albumin. Alternatively, said composition is free of mammalian derived proteins such as human serum albumin. Any such solution may provide sufficient stability to the Botulinum toxin complex. In other embodiments, said composition is free of any stabilizing agents.

The pharmaceutical composition in accordance with the present invention in one embodiment retains its potency substantially unchanged for a period of six months, one year, two years, three years and/or a four year period when stored at a temperature between about +26°C and about -20°C. Additionally, the indicated pharmaceutical compositions may have a potency or percent recovery of between about 20% and about 100% upon reconstitution.

"Cryoprotectant" refers to excipients which result in an active ingredient, i.e., a polypeptide of the invention in a reconstituted or aqueous solution pharmaceutical composition that has greater than about 20%, 30%, 40%, 50%, 60%, 70%, 80%, 90%, and up to about 100% of the toxicity that the biologically active polypeptide of the invention had prior to being freeze-dried in the pharmaceutical composition.

In one embodiment, the composition may contain a polyhydroxy compound, e.g. a polyalcohol as cryoprotectant. Examples of polyalcohols that might be used include, e.g., inositol, mannitol and other non-reducing alcohols. Some embodiments of the composition do not comprise a proteinaceous stabilizer, or do not contain trehalose or maltotriose or lactose or sucrose or related sugar or carbohydrate compounds which are sometimes used as cryoprotectants.

In one embodiment a preservative is part of the composition. In another embodiment, the composition of the present invention is free of any preservative.

The terms "preservative" and "preservatives" refer to a substance or a group of substances, respectively, which prevent the growth or survival of microorganisms, insects, bacteria or other contaminating organisms within said composition. Preservatives also prevent said composition from undesired chemical changes. Preservatives which can be used in the scope of this patent are all preservatives of the state of the art known to the skilled person. Examples of preservatives that might be used include, inter alia, e.g. benzylic alcohol, benzoic acid, sulphites (e.g. sulfur dioxide), formaldehyde, glutaraldehyde, diatomaceous earth, ethanol, methyl chloroisothiazolinone, butylated hydroxyanisole and/or butylated hydroxytoluene.

In one embodiment an analgesic is part of the composition, in another embodiment, an analgesic is administered before, during or after the treatment with the chemodenervating agent. In another embodiment, the composition of the present invention is free of an analgesic.

The term "analgesic" relates to analgesic drugs that act in various ways on the peripheral and central nervous systems and includes inter alia Paracetamol (acetaminophen), the nonsteroidal anti-inflammatory drugs (NSAIDs) such as the salicylates, narcotic drugs such as morphine, synthetic drugs with narcotic properties such as Tramadol, and various others. Also included is any compound with a local analgesic effect such as e.g. lidocaine, benzylic alcohol, benzoic acid and others.

In another embodiment, the polypeptide of this invention may be in the form of a lyophilisate.

The term "lyophilization" is used herein for a treatment of a solution containing the polypeptide, wherein this solution is frozen and dried until only the solid components of the composition are left over. The freeze-dried product of this treatment is therefore defined in this document as "lyophilisate".

As used herein, the term "reconstitution" and "reconstituted solution" is defined as the process of solubilization of said freeze-dried composition of the polypeptide of the invention. This can be done by adding the appropriate amount of sterile water, e.g. if all necessary components are already contained in the lyophilisate. Or, if this is not the case, it can be done e.g. by adding a sterile water-solution with the addition of components comprising e.g. a pH buffer, excipient, cryoprotectant, preservative, analgesic stabilizer or any combination thereof.

The solubilization is carried out in such a manner that the final "reconstitution" is directly or indirectly, i.e. for example after dilution, administrable to the patient.

In one embodiment the pharmaceutical composition of the invention is appropriate for
(a) treating a disease or condition which can be ameliorated by temporarily reducing muscle activity;
(b) treating a disease or condition which can be ameliorated by temporarily reducing glandular secretion;
(c) temporarily reducing muscle activity during an operation or in support of wound healing; and/or
for diagnosing any disease or condition of (a) to (c).

In one embodiment the composition of the invention is a pharmaceutical composition or a cosmetic composition.

In another embodiment, the composition of the invention is part of a diagnostic assay, e.g. a phrenic nerve-hemidiaphragm-assay, which allows diagnosing e.g. antibody levels in a patient blood-serum.

Treatments offered by the present invention may be directed at any of the following indications, most of which are described in detail in Dressler D (2000) (Botulinum Toxin Therapy. Thieme Verlag, Stuttgart, New York) for botulinum toxin:
■ dystonia
■ cranial dystonia
   - blepharospasm
   - oromandibular dystonia
      ○ jaw opening type
      ○ jaw closing type
   - bruxism
   - Meige syndrome
   - lingual dystonia
   - apraxia of eyelid opening
■ cervical dystonia
   - antecollis
   - retrocollis
   - laterocollis
   - torticollis
■ pharyngeal dystonia
■ laryngeal dystonia
   - spasmodic dysphonia/adductor type
   - spasmodic dysphonia/abductor type
   - spasmodic dyspnea
■ limb dystonia
   - arm dystonia
      ○ task specific dystonia
         ■ writer's cramp
         ■ musician's cramps
         ■ golfer's cramp
   - leg dystonia
      ○ thigh adduction, thigh abduction
      ○ knee flexion, knee extension
      ○ ankle flexion, ankle extension
      ○ equinovarus deformity
   - foot dystonia
      ○ striatal toe
      ○ toe flexion
      ○ toe extension
   - axial dystonia
      ○ pisa syndrome
      ○ belly dancer dystonia
   - segmental dystonia
   - hemidystonia
   - generalised dystonia
■ dystonia in lubag
■ dystonia in corticobasal degeneration
■ dystonia in lubag
■ tardive dystonia
■ dystonia in spinocerebellar ataxia
■ dystonia in Parkinson's disease
■ dystonia in Huntington's disease
■ dystonia in Hallervorden Spatz disease
■ dopa-induced dyskinesias/dopa-induced dystonia
■ tardive dyskinesias/tardive dystonia
■ paroxysmal dyskinesias/dystonias
   - kinesiogenic
   - non-kinesiogenic
   - action-induced
■ palatal myoclonus
■ myoclonus
■ myokymia
■ rigidity
■ benign muscle cramps
■ hereditary chin trembling
■ paradoxic jaw muscle activity
■ hemimasticatory spasms
■ hypertrophic branchial myopathy
■ maseteric hypertrophy
■ tibialis anterior hypertrophy
■ nystagmus
■ oscillopsia
■ hyperhydrosis
■ supranuclear gaze palsy
■ epilepsia partialis continua
■ planning of spasmodic torticollis operation
■ abductor vocal cord paralysis
■ recalcitant mutational dysphonia
■ upper oesophageal sphincter dysfunction
■ vocal fold granuloma
■ stuttering
■ Gilles de la Tourette syndrom
■ middle ear myoclonus
■ protective larynx closure
■ postlaryngectomy speech failure
■ protective ptosis
■ entropion
■ sphincter Odii dysfunction
■ pseudoachalasia
■ nonachalsia oesophageal motor disorders
■ vaginismus
■ postoperative immobilisation
■ tremor
■ genito-urinary diseases
   - bladder dysfunction
   - overactive bladder
      ○ urinary incontinence
      ○ urinary retention
      ○ spastic bladder
■ gastro-intestinal diseases
■ detrusor sphincter dyssynergia
■ bladder sphincter spasm
■ hemifacial spasm
■ reinnervation dyskinesias
■ cosmetic use
   - crow's feet
   - frowning
   - facial asymmetries
   - mentalis dimples
   - frontal lines
   - masseter lift
   - platysma
   - smoker's lines
   - marionette lines
■ stiff person syndrome
■ tetanus
■ prostate diseases
   - prostate hyperplasia
   - prostate cancer
■ adipositas treatment
■ infantile cerebral palsy
■ strabismus
■ mixed
■ paralytic
■ concomitant
■ after retinal detachment surgery
■ after cataract surgery
■ in aphakia
■ myositic strabismus
■ myopathic strabismus
■ dissociated vertical deviation
■ as an adjunct to strabismus surgery
■ esotropia
■ exotropia
■ achalasia
■ anal fissures
■ exocrine gland hyperactivity
■ Frey syndrome
■ Crocodile Tears syndrome
■ hyperhidrosis
   - axillar
   - palmar
   - plantar
■ rhinorrhea
■ relative hypersalivation
   - in stroke
   - in parkinson's
   - in amyotrophic lateral sclerosis
■ spastic conditions
   - in encephalitis and myelitis
      ○ autoimmune processes
         ■ multiple sclerosis
         ■ transverse myelitis
         ■ Devic syndrome
      ○ viral infections
      ○ bacterial infections
      ○ parasitic infections
      ○ fungal infections
   - in hereditary spastic paraparesis
   - postapoplectic syndrome
      ○ hemispheric infarction
      ○ brainstem infarction
      ○ myelon infarction
   - in central nervous system trauma
      ○ hemispheric lesions
      ○ brainstem lesions
      ○ myelon lesion
   - in central nervous system hemorrhage
      ○ intracerebral hemorrhage
      ○ subarachnoidal hemorrhage
      ○ subdural hemorrhage
      ○ intraspinal hemorrhage
   - in neoplasias
      ○ hemispheric tumors
      ○ brainstem tumors
      ○ myelon tumors
■ headache
   - migraine
   - tension headache
   - sinus headache
   - chronic headache
■ and/or hair loss.

The pharmaceutical composition of the present invention is administered, in one embodiment several times, in an effective amount for improving the patient's condition.

Typically, the dose administered to the patient will be up to about 1000 units, but in general will be below about 400 units per patient. In one embodiment the range lies between about 80 to about 400 units. These values are in one embodiment valid for adult patients. For children, the respective doses range from 25 to 800 and in another embodiment from 50 to 400 units.

While the above ranges relate to the maximum total doses, the dose range per muscle is in one embodiment within 3 to 6 units/kg body weight (b.w.), for small muscles 0,5 2 U/kg b.w., in another embodiment 0,1-1 U/kg b.w. Generally doses will not exceed 50 U per injection site and 100 U per muscle.

In one embodiment of the present invention the effective amount of botulinum toxin administered exceeds 500 U of the polypeptide of the invention in adults or exceeds 15 U/kg body weight in children.

While the above stated values are to be understood as a general guideline for administering the pharmaceutical composition of the present invention, it is, however, ultimately the physician who is responsible for the treatment who decides on both the quantity of toxin administered and the frequency of its administration.

The pharmaceutical composition of the present invention can be injected directly into the affected muscles. In order to find the appropriate injection site, several means exist which help the physician in order to find the same. Within the present invention, all methods for finding the best injection site are applicable, such as injection guided by electromyography (EMG), injection guided by palpation, injection guided by CT/MRI, as well as injection guided by sonography (ultra-sound). Among those methods, the latter is in one embodiment the method of choice when treating children. With respect to further details regarding the injection guided by sonography, we refer to Berweck "Sonography-guided injection of botulinum toxin A in children with cerebral palsy", Neuropediatric 2002 (33), 221-223.

The term "injection" is defined herein as any process, which allows the person skilled in the art to administer the active agent to the target site by penetrating the skin. An incomplete number of examples for "injections" are subcutaneous, intramuscular, intra-venous, intra-thecal, intra-arterial, etc..

In another embodiment, the composition is part of a diagnostic assay, e.g. a phrenic nerve-hemidiaphragm-assay, which allows to diagnose e.g. antibody levels in a patient blood-serum. Examples for such assays can be found in e.g. Göschel H, Wohlfarth K, Frevert J, Dengler R, Bigalke H. "Botulinum A toxin therapy: neutralizing and nonneutralizing antibodies--therapeutic consequences." Exp Neurol. 1997 Sep;147(1):96-102.

It is to be understood that the terminology used herein is for the purpose of describing particular embodiments only and is not intended to be limiting. It must be noted that, as used in the specification and the appended claims, the singular forms "a", "an" and "the" include plural referents unless the context clearly dictates otherwise.

The present invention is now further exemplified by way of the non-limited examples recited herein under.

### EXAMPLE

In the following example, Botox® and Merz research complex (prepared according to Methods in Molecular Biology, Vol. 145 (2000), p. 27-39 with a final size exclusion chromatography step instead of crystallization) were investigated as to the stability of the Botulinum toxin complex when being formulated, diluted, freeze-dried and reconstituted in different media.

Firstly, Botox® was reconstituted with different solutions, i.e. ultrapure water and 0.9% saline, followed by ultracentrifugational sedimentation speed analysis (UC analysis). In a second series, BoNT/A research complex was diluted to a final concentration of 50 ng/mL with either 0.94% sucrose / 0.2% HSA or with 0.9% NaCl /0.5% HSA followed by UC analysis.

If reconstituted with NaCl, the fraction of the intact Botulinum toxin complex within Botox® is determined 8.4%. Reconstitution with water results in 32.8% Botulinum toxin complex of ∼500 kD.

The dilution of research grade BoNT/A 900 kDa complex with 0.94% sucrose /0.5% HSA to a final concentration of 50 ng/mL yields virtually 100% Botulinum toxin complex, i.e. ∼500 kD particles. On the other hand, the same complex diluted with 0.9% Nacl / 0.5% HSA results in solely released 150 kD, i.e. "free" neurotoxic component which was no longer associated complex proteins.

Thus, the Botulinum toxin complex integrity seems to be fundamentally affected by the salt concentration. The presence of 0.9% NaCl leads to dissociation of the complex during dilution of a highly concentrated stock solution and also results in a release of the neurotoxic component from the complex of Botox^{®} already at reconstitution, i.e. prior to administration / injection. This finding is surprising in view of the common belief and the respective marketing efforts starting that Botox^{®} contains the intact 900 kD Botulinum toxin complex.

## Claims

1. A salt-free pharmaceutical composition comprising an intact Botulinum toxin complex and at least one pharmaceutically acceptable excipient.

2. The pharmaceutical composition of claim 1, wherein the excipient is selected from one or more members of the group consisting of a saccharide, a polysaccharide, a di-saccharide, a mono-saccharide, amylopectin, glucopyranose, starch, hydroxyethyl starch, hydroxymethyl starch, sucrose, PEG.

3. The pharmaceutical composition of claim 1 or 2, wherein the excipient is selected from one or more members of the group consisting of albumin, human serum albumin, bovine serum albumin, recombinant albumin, cryoprotectant, preservative , analgesic and sterile distilled water.

4. The pharmaceutical composition of any of the preceding claims, wherein the botulinum toxin complex is selected from the group consisting of botulinum toxin types A, B, C1, D, E, F and G.

5. The pharmaceutical composition of claim 4, wherein the botulinum toxin is botulinum toxin type A.

6. A lyophilized or vacuum dried salt-free pharmaceutical composition consisting essentially of a high molecular weight polysaccharide and a botulinum toxin complex.

7. The pharmaceutical composition according to any of the preceding claims in lyophilized, vacuum dried or liquid injectable form.

8. A method for stabilizing a Botulinum toxin complex against denaturation or aggregation, the method comprising the step of formulating a Botulinum neurotoxin complex without any salt.

9. A method for the preparation of a salt-free pharmaceutical composition of any of claims 1 to 7 comprising the admixture of a Botulinum toxin complex and at least one pharmaceutically acceptable excipient, wherein said Botulinum toxin complex is not subjected to lyophylisation prior to said step of admixing.

10. A method of using the pharmaceutical composition of any of the claims 1 to 7, or the pharmaceutical composition prepared by the method of claim 8 or 9 for the treatment of a disease or condition caused by or associated with hyperactive cholinergic enervation of muscles or exocrine glands comprising the step of local administration of said pharmaceutical composition to a patient in need thereof.

11. A pharmaceutical composition of any of the claims 1 to 7, or a pharmaceutical composition prepared by the method of claim 8 or 9 for use in the treatment of a disease or condition caused by or associated with hyperactive cholinergic enervations of muscles or exocrine glands in a patient in need thereof.

12. Use of the pharmaceutical composition of any of the claims 1 to 7, or the pharmaceutical composition prepared by the method of claim 8 or 9 for cosmetic treatment.

## Amended claims

### Amended claims in accordance with Rule 137(2) EPC.

**1.** A salt-free pharmaceutical composition comprising an intact Botulinum toxin complex and at least one pharmaceutically acceptable excipient.

**2.** The pharmaceutical composition of claim 1, wherein the excipient is selected from one or more members of the group consisting of a saccharide, a polysaccharide, a di-saccharide, a mono-saccharide, amylopectin, glucopyranose, starch, hydroxyethyl starch, hydroxymethyl starch, sucrose, PEG.

**3.** The pharmaceutical composition of claim 1 or 2, wherein the excipient is selected from one or more members of the group consisting of albumin, human serum albumin, bovine serum albumin, recombinant albumin, cryoprotectant, preservative , analgesic and sterile distilled water.

**4.** The pharmaceutical composition of any of the preceding claims, wherein the botulinum toxin complex is selected from the group consisting of botulinum toxin types A, B, C1, D, E, F and G.

**5.** The pharmaceutical composition of claim 4, wherein the botulinum toxin is botulinum toxin type A.

**6.** A lyophilized or vacuum dried salt-free pharmaceutical composition consisting essentially of a high molecular weight polysaccharide and a botulinum toxin complex.

**7.** The pharmaceutical composition according to any of the preceding claims in lyophilized, vacuum dried or liquid injectable form.

**8.** A method for stabilizing a Botulinum toxin complex against denaturation or aggregation, the method comprising the step of formulating a Botulinum neurotoxin complex without any salt.

**9.** A method for the preparation of a salt-free pharmaceutical composition of any of claims 1 to 7 comprising the admixture of a Botulinum toxin complex and at least one pharmaceutically acceptable excipient, wherein said Botulinum toxin complex is not subjected to lyophylisation prior to said step of admixing.

**10.** A method of using the pharmaceutical composition of any of the claims 1 to 7, or the pharmaceutical composition prepared by the method of claim 8 or 9 for use in the treatment of a disease or condition caused by or associated with hyperactive cholinergic enervation of muscles or exocrine glands comprising the step of local administration of said pharmaceutical composition to a patient in need thereof.

**11.** A pharmaceutical composition of any of the claims 1 to 7, or a pharmaceutical composition prepared by the method of claim 8 or 9 for use in the treatment of a disease or condition caused by or associated with hyperactive cholinergic enervations of muscles or exocrine glands in a patient in need thereof.

**12.** Use of the pharmaceutical composition of any of the claims 1 to 7, or the pharmaceutical composition prepared by the method of claim 8 or 9 for cosmetic treatment.
